# FASCICULE DE BREVET EUROPEEN

(11) **EP 3 589 946 B1**
(45) Date de publication et mention de la délivrance du brevet: **09.12.2026**
(21) Numéro de dépôt: 18707921.5
(22) Date de dépôt: 02.03.2018
(51) Int. Cl.: G01N 33/00, G01N 21/27, G01N 21/55, G01N 21/552, G01N 21/61, G01N 21/93

(54) **PROCEDE DE CALIBRATION D'UN NEZ ELECTRONIQUE**
VERFAHREN ZUR KALIBRIERUNG EINER ELEKTRONISCHEN NASE
METHOD FOR CALIBRATING AN ELECTRONIC NOSE

(30) Priorité: 03.03.2017 FR 1751751
(43) Date de publication de la demande: 08.01.2020
(73) Titulaire: Commissariat à l'Energie Atomique et aux Energies Alternatives, 75015 Paris (FR); Centre National de la Recherche Scientifique, 75016 Paris (FR); Université Grenoble Alpes, 38400 Saint-Martin-d'Hères (FR); Olfaktion, 38000 Grenoble (FR)
(72) Inventeur: HERRIER, Cyril, 38000 Grenoble (FR); HOU-BROUTIN, Yanxia, 38850 Bilieu (FR); GALLAT, François-Xavier, 38000 Grenoble (FR); LIVACHE, Thierry, 38560 Jarrie (FR); ROUSSELLE, Tristan, 38100 Grenoble (FR)
(74) Mandataire: Gevers & Orès
(86) Numéro de dépôt international: PCT/EP2018/055233
(87) Numéro de publication internationale: WO 2018/158458

(56) Documents cités:
- WO-A1-2015/102090
- WO-A2-2007/003674
- CN-B- 103 500 770
- JP-A- 2017 044 577
- US-A1- 2009 046 292
- ISABEL M PÀREZ DE VARGAS-SANSALVADOR ET AL: "Compact optical instrument for simultaneous determination of oxygen and carbon dioxide", MICROCHIMICA ACTA ; AN INTERNATIONAL JOURNAL ON MICRO AND TRACEANALYSIS, SPRINGER-VERLAG, VI, vol. 172, no. 3 - 4, 17 December 2010 (2010-12-17), pages 455 - 464, XP019885628, ISSN: 1436-5073, DOI: 10.1007/S00604-010-0520-0
- ISABEL M PÃ CR REZ DE VARGAS-SANSALVADOR ET AL: "Compact optical instrument for simultaneous determination of oxygen and carbon dioxide", MICROCHIMICA ACTA ; AN INTERNATIONAL JOURNAL ON MICRO AND TRACEANALYSIS, SPRINGER-VERLAG, VI, vol. 172, no. 3 - 4, 17 December 2010 (2010-12-17), pages 455 - 464, XP019885628, ISSN: 1436-5073, DOI: 10.1007/S00604-010-0520-0
- PALMA ET AL: "Microcontroller-based portable instrument for stabilised optical oxygen sensor", SENSORS AND ACTUATORS B: CHEMICAL: INTERNATIONAL JOURNAL DEVOTED TO RESEARCH AND DEVELOPMENT OF PHYSICAL AND CHEMICAL TRANSDU, ELSEVIER BV, NL, vol. 121, no. 2, 3 February 2007 (2007-02-03), pages 629 - 638, XP005872249, ISSN: 0925-4005, DOI: 10.1016/J.SNB.2006.03.056
- XIUTAO LOU ET AL: "Temperature-Corrected Oxygen Detection Based on Multi-Mode Diode Laser Correlation Spectroscopy", JOURNAL OF SPECTROSCOPY, vol. 32, no. 30, 1 January 2013 (2013-01-01), pages 6090 - 8, XP055152166, ISSN: 2314-4920, DOI: 10.1155/2013/524071

## Description

L'invention concerne un procédé de calibration d'un nez électronique.

Un nez électronique comprend généralement plusieurs capteurs, visant à reconnaître la présence d'un composé cible, par exemple un analyte chimique ou biologique, dans un échantillon gazeux.

Les capteurs ne sont généralement pas spécifiques à un composé cible particulier. Aussi, dans une application donnée on réalise généralement une comparaison des données fournies par les différents capteurs du nez électronique, lesquels fournissent une empreinte de reconnaissance, à des données de références, par exemple issues d'un apprentissage préalable pour le composé cible en question.

Une technique connue pour obtenir, en utilisation, une empreinte de reconnaissance est l'imagerie par résonance plasmon de surface (plus connu sous l'acronyme SPR pour « Surface Plasmon Resonance »). Cette technique permet de détecter un changement local d'indice optique (indice optique = indice de réfraction) qui caractérise l'interaction du composé cible avec chaque capteur du nez électronique.

Cependant, dans la mesure où l'on ne connaît a priori pas les affinités chimiques de chaque capteur du nez électronique vis-à-vis d'un composé cible donné et que seule l'empreinte de l'ensemble des capteurs est pris en compte pour la reconnaissance du composé cible, il est nécessaire que chaque capteur réponde de façon reproductible l'un par rapport à l'autre et d'une expérience à l'autre. De même, il est nécessaire que des nez électroniques différents, à savoir notamment issus de lots de fabrication différents, puissent donner des réponses reproductibles.

Ces mêmes difficultés de reproductibilité sont rencontrées avec des capteurs conçus pour être spécifiques à un composé cible particulier.

A défaut, il n'est pas possible d'obtenir une empreinte de reconnaissance fiable, apte à être comparée aux données de référence.

En effet, bien que tout le soin nécessaire soit apporté à la fabrication d'un nez électronique, les capteurs présentent des légères différences par rapport à leur conception idéale.

Il existe déjà plusieurs techniques pour réaliser la calibration d'un nez électronique.

Une première technique est proposée dans la documentation Permapure du 14 juin 2016, intitulée « Gas Sensor Calibration » accessible sur le site http://www.permapure.com/wp-content/uploads/2013/01/calibration.pdf, issue du livre « Air Monitoring for Toxic exposure », Henry J. McDermott, 2ème édition, 2004, John Wiley & Sons Inc., pp. 161-173 (D1).

Dans cette technique, la calibration s'effectue en injectant un gaz comportant un composé organique de référence.

Une deuxième technique consiste à utiliser un modèle de prédiction après injection d'un composé organique de référence à différentes concentrations. C'est ce qui est proposé par Tian & al., « On-line calibration of semiconductor gas sensors based on, prediction model », J. of computers, vol. 8, p. 2204, Septembre 2013 (D2).

Pour ces deux techniques, le stimulus commun à l'ensemble des capteurs est donc basé sur un composé organique de référence. On parle de calibration chimique.

De plus et en pratique, si l'on souhaite obtenir un nez électronique versatile, on prévoit alors plusieurs composés organiques de référence.

Toutefois, avec ces techniques, il arrive, selon la concentration du composé organique de référence, ou en passant d'un composé organique de référence à un autre, qu'il y ait des affinités distinctes des différents capteurs du nez électronique.

Cela est alors néfaste pour la qualité de la calibration.

Par ailleurs, ce type de calibration n'est pas très pratique puisqu'il est parfois nécessaire d'avoir avec soi les différents composés organiques de référence.

D'autres méthodes de calibration existent et sont mis en œuvre notamment par Vargas-Sansalvador et al. dans « Compact optical instrument for simultaneous determination of oxygen and carbon dioxide », Michrochimica Acta ; An International Journal on Micro and Traceanalysis, Springer-Verlag, VI, vol. 172, p.455-464, Décembre 2010, par US-A1-2009/046292 ou CN-B-103 500 770.

Un objectif de l'invention est ainsi de proposer un procédé de calibration d'un nez électronique ne présentant pas l'un au moins des inconvénients précités.

Pour atteindre cet objectif, l'invention propose un procédé de calibration d'un nez électronique selon la revendication 1.

Il convient de rappeler que les capteurs, étant donné leur sensibilité à une variation d'indice optique local de par les procédés de lecture utilisés, sont intrinsèquement sensibles à la température et/ou à la pression. Ceci est considéré par l'homme de l'art comme un inconvénient.

Dans le cadre de l'invention, on comprend donc qu'il est proposé d'utiliser cette sensibilité pour effectuer une calibration physique.

Le procédé selon l'invention pourra comprendre l'une au moins des caractéristiques suivantes, prise seule ou en combinaison :
- préalablement à l'étape a), on détermine la pression P₀ et/ou la température T₀ du milieu gazeux d'intérêt ;
- la mesure réalisée à l'étape b) ou d), par exemple une mesure de réflectivité ou de transmittivité, s'effectue sur une durée comprise entre 0,1s et 60mn, de préférence entre 1s et 10mn, puis moyennée ;
- avant de mettre en œuvre l'étape e), on répète N fois les étapes c) et d), avec N un entier naturel supérieur ou égal à 1, de sorte que la pression et/ou la température du milieu gazeux d'intérêt soit différente d'une pression et/ou d'une température du milieu gazeux d'intérêt pour lequel une mesure a déjà été effectuée ;
- au cours de l'étape c), on modifie la pression et/ou la température du milieu gazeux d'intérêt vers une autre valeur connue ;
- à l'étape c), on modifie la pression du milieu gazeux d'intérêt d'une valeur comprise entre +10mbar et +2bar, de préférence entre +50mbar et +150mbar ou d'une valeur comprise entre -10mbar et -900mbar, de préférence entre -50mbar et -150mbar ; et/ou on modifie la température du milieu gazeux d'une valeur comprise entre +1°C et +100°C, de préférence entre +5°C et +15°C ou entre -1°C et -50°C, de préférence entre -5°C et -15°C ;
- juste avant l'étape e), on met en œuvre une étape additionnelle consistant à modifier la pression et/ou la température du milieu gazeux d'intérêt à la pression initiale (P₀) et/ou la température initiale (T₀) ;
- le capteur optique est choisi parmi un capteur à effet plasmon, par exemple sur surface plane, fibre optique ou nanocavités ou un capteur susceptible de fonctionner par réfractométrie, par exemple un capteur à résonateur.

Pour atteindre ce même objectif, l'invention propose également un procédé de calibration d'un nez électronique selon la revendication 9.

Ce procédé selon l'invention pourra comprendre l'une au moins des caractéristiques suivantes, prise seule ou en combinaison :
- préalablement à l'étape A), on règle la pression et/ou la température du milieu gazeux d'intérêt à une valeur prédéterminée ;
- la mesure réalisée à l'étape C) ou E), par exemple une mesure de réflectivité ou de transmittivité, s'effectue sur une durée comprise entre 0,1s et 60mn, de préférence entre 1s et 10mn, puis moyennée ;
- avant de mettre en œuvre l'étape F), on répète N fois les étapes D) et E), avec N un entier naturel supérieur ou égal à 1, de sorte que la pression ou selon le cas, de la température du milieu gazeux d'intérêt soit différente d'une pression ou selon le cas d'une température du milieu gazeux d'intérêt pour lequel une mesure a déjà été effectuée ;
- à l'étape D) on modifie la pression du milieu gazeux d'une valeur comprise entre +10mbar et + 2bar, de préférence entre +50mbar et +150mbar ou d'une valeur comprise entre -10mbar et -900mbar, de préférence entre -50mbar et -150mbar ; et/ou on modifie la température du milieu gazeux d'une valeur comprise entre +1°C et +100°C, de préférence entre +5°C et +15°C, ou entre -1°C et -50°C, de préférence entre -5°C et -15°C ;
- juste avant l'étape F), on met en œuvre une étape additionnelle consistant à modifier la pression et/ou la température du milieu gazeux d'intérêt à la pression initiale (P₀) et/ou la température initiale (T₀) ;
- le capteur optique est choisi parmi un capteur à effet plasmon, par exemple sur surface plane, fibre optique ou nanocavités, ou un capteur susceptible de fonctionner par réfractométrie, par exemple un capteur à résonateur.

D'autres caractéristiques, buts et avantages de l'invention ressortiront à la lecture de la description faite en référence aux figures annexées données à titre d'exemple, et dans lesquelles :
- la figure 1 représente une installation envisageable pour mettre en œuvre un procédé conforme à l'invention, basé sur une mesure en réflectivité et une évolution de pression du milieu gazeux associé au nez électronique ;
- la figure 2 est une image typique générée par l'installation suscitée sur laquelle est visible les capteurs du nez électronique ;
- la figure 3 représente des résultats de mesure de réflectivité effectuée avec l'installation des figures 1 et 2, avec de l'air sec comme milieu gazeux ;
- la figure 4, qui comporte les figures 4(a) à 4(c), représente un cas d'application susceptible d'être effectué avec l'installation des figures 1 et 2, avec un milieu gazeux comportant de l'air (sec) et de l'éthanol, servant d'analyte ;
- la figure 5 représente une variante de l'installation des figures 1 et 2 pour mettre en œuvre un procédé conforme à l'invention, basé sur une mesure en réflectivité et une évolution de température du milieu gazeux associé au nez électronique ;
- la figure 6 représente une autre variante de l'installation des figures 1 et 2 pour mettre en œuvre un procédé conforme à l'invention, basé sur une mesure en transmittivité et une évolution de pression et de température du milieu gazeux associé au nez électronique.

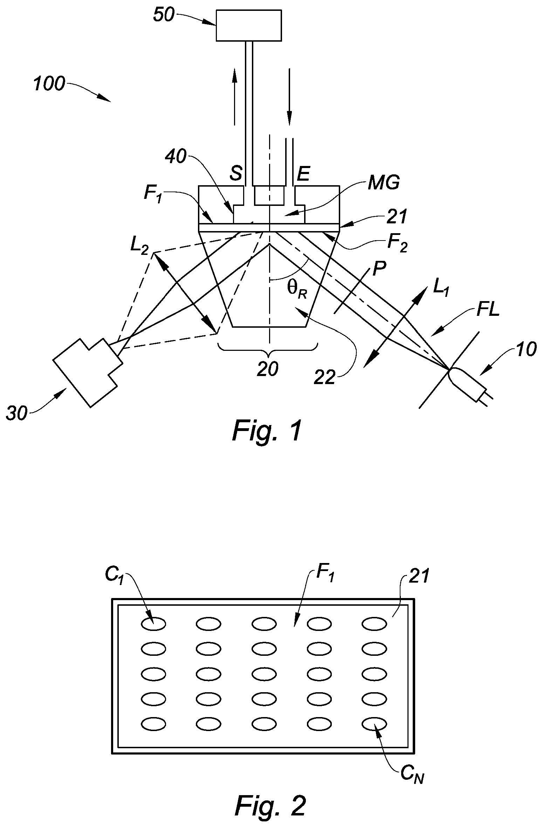

La figure 1 représente un exemple d'installation expérimentale 100 permettant de mettre œuvre le procédé de calibration d'un nez électronique selon l'invention.

Cette installation expérimentale 100 comporte une source de lumière 10, par exemple une DEL, capable d'émettre une longueur d'onde donnée, un nez électronique 20 et une sonde optique 30, par exemple une caméra CCD. Une lentille L1 et un polariseur P peuvent être prévus entre la source de lumière 10 et le nez électronique 20. Une lentille L2 peut également être prévue entre le nez électronique 20 et la sonde optique 30.

On note que la sonde optique 30 est agencée du même côté de la couche métallique 21 que la source de lumière 20. Cette installation expérimentale 100 permet donc d'effectuer des mesures en réflexion.

Le nez électronique 20 comprend une couche métallique 21, en l'occurrence en Or (Au), plane.

Le nez électronique 20 comprend également une pluralité de capteurs C₁, ..., C_{N} agencés sur une première face F1 de ladite couche métallique 21 de sorte que ladite première face F1 de la couche métallique 21 et lesdits capteurs soient en contact avec un milieu gazeux, par nature diélectrique. Comme pour tout nez électronique, il y a au moins deux capteurs optiques de sensibilité chimique différente parmi la pluralité de capteurs optiques du nez électronique.

Le nez électronique 20 comprend également un support 22 pour ladite couche métallique 21. Le support 22 est agencé contre une deuxième face F2 de la couche métallique 21, ladite deuxième face F2 étant opposée à ladite première face F1. De manière générale, le support 22 est choisi en un matériau diélectrique, transparent à la longueur d'onde que la source de lumière 10 est destinée à émettre et présentant un indice optique *n_{S}* supérieur à l'indice optique *n_{G}* du milieu gazeux (indice optique = indice de réfaction). Dans le cas d'espèce, il s'agit d'un prisme, réalisé en verre. Une autre couche métallique (non représentée) de faible épaisseur, par exemple réalisée en Chrome (Cr), est prévue entre la deuxième face F2 de la couche métallique 21 et le support 22 pour assurer l'accroche de la couche métallique 21 sur le support 22.

Une telle installation 100 permet de générer une résonance plasmon au niveau de la première face de la couche métallique 21 qui est en contact avec le milieu gazeux. Plus précisément, si l'on définit l'angle d'incidence entre la direction de propagation du faisceau lumineux FL et la normale à la couche métallique 21, on peut définir la relation suivante : ${n}_{S} sin \left({θ}_{R}\right) = \sqrt{\frac{{ε}_{m} {ε}_{g}}{{ε}_{m} + {ε}_{g}}}$ (R1) où :
*n_{S}* est l'indice de réfaction du support 22,
*εₘ* est la permittivité du métal formant la couche métallique 21,
*ε_{g}* est la permittivité du milieu gazeux MG, *et*
*θ_{R}* est l'angle d'incidence de résonance plasmon.

La relation (R1) fait implicitement intervenir la longueur d'onde du faisceau lumineux FL émis par la source optique 10. En effet et par exemple, l'indice optique *n_{G}* du milieu gazeux MG et donc sa permittivité *ε_{g}* dépendent de la longueur d'onde.

Ainsi, pour une longueur d'onde donnée du faisceau lumineux FL, pour une couche métallique 21 donnée (nature du matériau métallique) et pour un milieu gazeux MG donné, il existe un angle d'incidence *θ_{R}* tel que défini précédemment, qui permet d'obtenir la résonance plasmon.

Cette installation expérimentale 100 reprend donc les caractéristiques de la configuration de Kretschmann.

La fabrication d'une telle configuration de Kretschmann est connue de l'homme du métier et n'est donc pas précisée. Toutefois, on pourra se référer à l'article de Guedon & al. intitulé « Characterization and Optimization of a Real-Time, Parallel, Label-Free, Polypyrolle based DNA Sensor by Surface Plasmon Imaging" , Anal. Chem., 2000, vol. 72, pp. 6003-6009 pour plus d'informations.

La résonance plasmon permet dans ce cas d'induire une onde plasmon à l'interface entre la couche métallique et le milieu gazeux, dont l'amplitude permet d'observer avec une bonne sensibilité des variations locales de propriétés optiques, telles qu'une variation d'indice optique ou une variation de réflectivité. Aussi, dans le cas de la résonance plasmon, le signal délivré par les capteurs C₁, ..., C_{N} pourra notamment être représentatif d'une variation de réflectivité.

Le demandeur a pu s'apercevoir qu'il était envisageable, avec l'installation expérimentale 100 de réaliser une calibration, en l'occurrence relative, des capteurs, en faisant varier la pression et/ou la température du milieu gazeux MG.

Par calibration relative, il faut comprendre une calibration des capteurs les uns par rapport aux autres, et plus précisément en choisissant un capteur comme référence ou une combinaison de capteurs comme référence, les autres capteurs étant alors calibrés par rapport à ce capteur de référence ou cette combinaison de capteurs comme référence. On comprend donc que le capteur de référence est un capteur choisi parmi les différents capteurs optiques du nez électronique ou que, de manière similaire, la combinaison de capteurs de référence est un ensemble de capteurs choisis parmi les différents capteurs optiques du nez électronique. Dans cette calibration relative, il y a toujours un stimulus commun, car cela est nécessaire pour s'assurer d'une réponse identique de l'ensemble des capteurs. Ce stimulus commun est en l'occurrence la pression et/ou la température du milieu gazeux MG. La connaissance exacte (valeur) du stimulus commun n'est en revanche pas nécessaire pour effectuer une calibration relative.

En revanche, cette calibration relative ne permet pas de calibrer le nez électronique pour s'assurer qu'en utilisation (c'est-à-dire après calibration et pour détecter par exemple la présence d'un composé cible particulier), l'utilisation d'un dispositif du type de la configuration de Kretschmann fournira des valeurs absolues d'une variation d'indice optique locale permettant de caractériser ce composé cible particulier.

Toutefois, une calibration chimique peut être effectuée en amont, par exemple en usine. Cette calibration chimique peut notamment s'effectuer par une technique connue, telle que celle décrite dans le document D1 ou D2.

Dans ce cas, la calibration relative effectuée dans le cadre de l'invention permettra assurément de calibrer le nez électronique pour qu'il soit utilisable.

L'installation expérimentale 100 a été plus précisément conçue pour s'assurer d'un stimulus commun en pression. A cet effet, la couche métallique 21 et ses capteurs sont logés dans une chambre 40 comprenant une entrée E et une sortie S. La sortie S est reliée à une pompe 50 permettant d'alimenter la chambre avec un flux gazeux parfaitement contrôlé. Cela signifie que le débit de gaz est contrôlé, à savoir connu, pour obtenir un flux gazeux laminaire dans la chambre. Il y a en effet un lien entre la pression et la vitesse du flux gazeux. Typiquement, on peut s'appuyer sur la relation de Bernoulli dans le cas d'un fluide newtonien.

Un premier procédé selon l'invention est un procédé de calibration d'un nez électronique, ledit nez électronique 20 comportant une pluralité de capteurs optiques agencés sur une surface et aptes à être en contact avec un milieu gazeux d'intérêt, lesdits capteurs optiques étant aptes à délivrer un signal représentatif de l'indice optique local du milieu gazeux d'intérêt lorsqu'ils sont excités par des photons, le procédé étant caractérisé en ce qu'il comprend les étapes suivantes, après avoir placé le nez électronique dans un milieu gazeux d'intérêt à la pression initiale P₀ et la température initiale T₀ :
a) émettre des photons en direction des capteurs optiques de façon à exciter lesdits capteurs;
b) mesurer le signal délivré par chacun des capteurs optiques, cette mesure fournissant autant de réponses qu'il y a de capteurs optiques ;
c) modifier la pression et/ou la température du milieu gazeux d'intérêt ;
d) répéter l'étape b); et
e) pour chaque capteur, déterminer un facteur correctif tel qu'une variation du signal entre les étapes d) et b) corrigée par ledit facteur correctif soit égale ou sensiblement égale à une variation du signal entre ces mêmes étapes pour une référence, cette référence étant fournie par un capteur optique de référence ou une combinaison de capteurs optiques de référence.

Ce premier procédé de calibration permet de mettre en œuvre une calibration relative.

En effet, une fois que les facteurs correctifs pour les différents capteurs sont déterminés, la calibration du nez électronique est faite. Il ne reste alors qu'à la prendre en compte avant de débuter une mesure effective avec ce nez électronique.On présente ci-dessous un exemple, dans lequel ce premier procédé est mis en œuvre avec l'installation expérimentale 100.

La figure 2 est une vue de la couche métallique 21 et de ses capteurs C₁, ..., C_{N}.

La couche métallique 21, réalisée en Or, présente une permittivité complexe *εₘ*, à la longueur d'onde de 632nm, qui s'exprime *εₘ* = *εᵣ + i*εᵢ =* -11,6 + i*1,5 (avec i² = -1).

Par ailleurs, les capteurs optiques sont tous formés par la technique proposée par Hou & al., « Continuous evolution profiles for electronic-tongue-based analysis », Angewandte Chem. Int. Ed. 2012, vol. 51, pp.10394-10398 ; avec du décanthiol par exemple Les capteurs optiques obtenus après fonctionnalisation de leur surface ont alors tous une forme ronde.

On peut effectuer les mesures pour l'ensemble des capteurs. Néanmoins, dans le seul souci de la démonstration, il a été choisi ici de sélectionner seulement quatre d'entre eux. Cela peut se faire aisément en prévoyant un masque pour couvrir les capteurs pour lesquels on ne souhaite pas obtenir de réponse lors de la calibration.

Le milieu gazeux MG est de l'air sec.

La pression et la température de l'air sec de la chambre dans laquelle l'expérience est réalisée est telle que la pression initiale est de P₀ = 1,063 bar et la température T₀ est telle que T₀ = 25°C.

Pour rappel, dans le but d'obtenir une calibration relative, il n'est pas nécessaire de connaître ces données T₀, P₀. Ceci est en revanche important pour réaliser une calibration chimique.

Par ailleurs, ces valeurs permettent, par la relation R1, de calculer l'angle d'incidence théorique permettant d'obtenir la résonance plasmon.

La longueur d'onde λ du faisceau lumineux FL est telle que À = 632nm.

Dans ces conditions (T₀, P₀ et λ), la permittivité *ε_{g}*/*ε₀* statique relative du milieu gazeux est telle que *ε_{g}*/*ε₀* = 1,00058986 où *ε₀* est la permittivité du vide.

Par ailleurs et comme déjà indiqué, le support 22 est un prisme, correctement orienté, réalisé en verre. Son indice optique est de *n_{S} =* 1,51.

De ces différentes valeurs, on déduit alors que l'angle d'incidence *θ_{R}* tel que défini précédemment qui permet d'obtenir la résonance plasmon, conformément à la relation (R1), est de *θ_{R}* = 43°.

Il convient de noter, qu'en variante, on peut chercher cet angle expérimentalement.

On peut alors obtenir, à la pression P₀, la réponse en réflectivité de chacun des quatre capteurs sélectionnés. L'acquisition de la variation de réflectivité pour chacun de ces capteurs s'effectue en l'occurrence sur plusieurs minutes afin d'obtenir, pour chaque capteur, un certain nombre de valeurs qui sont ensuite avantageusement moyennées afin d'améliorer la précision de la mesure.

Pour mettre en œuvre l'étape qui suit, on réalise un saut de pression, en l'occurrence positif, de 100mbar pour régler la pression à une valeur P1 = 1,163 mbar. Dans le même temps, la température qui est celle de la chambre n'a pas changée.

Dans cet exemple, il a été choisi de répéter les étapes b) et c) sept fois afin de définir huit paliers de pression.

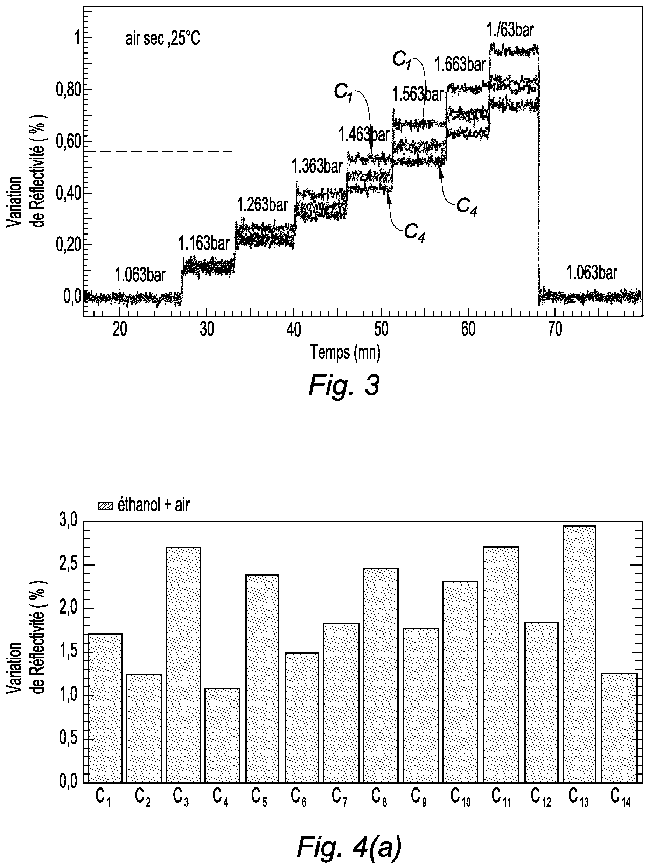

Les résultats de mesure en réflectivité sont représentés sur la figure 3 (signaux délivrés par les capteurs optiques C₁, ..., C_{N}). Cette figure 3 fournit l'évolution de la variation de réflectivité (%) dans le temps et pour chacun des quatre capteurs sélectionnés. La réflectivité (%) est définie par le rapport de l'intensité du faisceau lumineux reçu par la sonde optique sur l'intensité du faisceau lumineux émis par la source optique.

Dans la mesure où, dans le temps, plusieurs paliers de pression du milieu gazeux sont mis en œuvre, on peut observer que la réflectivité qui est mesurée se présente également sous la forme de paliers.

Ces résultats démontrent qu'il est tout à fait possible, avec un appareillage fonctionnant par résonance plasmon, de mesurer l'influence de la pression du milieu gazeux, avec la pression de ce milieu gazeux en tant que stimulus commun.

Ces résultats montrent par ailleurs la nécessité d'effectuer une calibration des différents capteurs puisqu'on constate une différence de réponse en réflectivité de chacun des capteurs lorsque la pression n'est plus la pression de référence P₀ pour laquelle l'installation expérimentale a initialement été préparée. En effet, si les capteurs fournissent des réflectivités différentes dans des conditions identiques (température et pression du milieu gazeux, longueur d'onde, notamment pour définir la permittivité du milieu gazeux) cela signifie que chacun des capteurs ne voient pas le même angle de résonance plasmon (cf. relation R1), ou qu'ils ont une sensibilité variable liée à, par exemple, la nature du composé formant le capteur, autrement dit qu'ils sont décalés les uns par rapport aux autres par rapport au pic de résonance plasmon.

C'est pourquoi, une fois les résultats de la figure 3 obtenus, on met en œuvre l'étape e).

A cet effet, on a en l'occurrence choisi comme référence un capteur, pour lequel on considère que la variation de réflectivité est correcte, pour l'ensemble des paliers de pression.

Pour chacun des autres capteurs, et à chaque palier de pression, on a alors déterminé un facteur correctif tel qu'une différence du signal entre les étapes d) et b) (différence de variation de réflectivité ici) soit égale ou sensiblement égale à une variation de la réflectivité du capteur de référence.

Par exemple, sur la figure 3 à la pression de 1,463 bar, le capteur de référence, C1, indique une variation de réflectivité mesurée de 0,54%, considérée correcte et le capteur C4 une variation de réflectivité mesurée de 0,42%. Pour le capteur C4, le facteur correctif est de 54/42 pour obtenir une variation de réflectivité corrigée du capteur C4, égale à la variation de réflectivité du capteur de référence, soit 0,54%.

Si une calibration chimique a été préalablement effectuée (par exemple par une méthode connue, en particulier en usine), on peut alors s'assurer que la calibration relative effectuée comme proposée précédemment permet de calibrer correctement le nez électronique car dans ce cas, on est certain que le capteur de référence fournit des valeurs correctes.

Dans l'exemple fourni et conduisant à la figure 3, le saut de pression est parfaitement déterminé, ce qui permet de connaître la pression modifiée après la mise en œuvre de l'étape d).

Il convient toutefois de noter que, dans ce procédé de calibration relative, il importe peu de connaître avec exactitude le saut de pression effectué à l'étape c), car la correction ne s'appuie pas sur la connaissance de ce saut de pression. Ce qui importe est que la gamme de pression soit en cohérence avec le domaine de travail du nez électronique.

Dans le cadre de l'invention, on peut envisager de mettre en œuvre un deuxième procédé de calibration d'un nez électronique, permettant également de réaliser une calibration relative.

Plus précisément, il s'agit d'un procédé de calibration d'un nez électronique, ledit nez électronique comportant une pluralité de capteurs optiques agencés sur une surface et aptes à être en contact avec un milieu gazeux d'intérêt, lesdits capteurs optiques étant aptes à délivrer un signal représentatif de l'indice optique local du milieu gazeux d'intérêt lorsqu'ils sont excités par des photons, le procédé étant caractérisé en ce qu'il comprend les étapes suivantes, après avoir placé le nez électronique dans un milieu gazeux d'intérêt à la pression initiale P₀ et la température initiale T₀ :
A) déterminer la pression initiale P₀ et la température initiale T₀ du milieu gazeux d'intérêt ;
B) émettre des photons en direction des capteurs optiques de façon à exciter lesdits capteurs ;
C) mesurer le signal délivré par chacun des capteurs optiques, cette mesure fournissant autant de réponses qu'il y a de capteurs optiques ;
D) modifier la pression et/ou la température du milieu gazeux d'intérêt vers une autre ou des autres valeurs connues ;
E) répéter l'étape C); et
F) pour chaque capteur, calculer l'évolution de l'indice optique du milieu gazeux d'intérêt à l'aide des mesures réalisées aux étapes C) et E).

Les étapes B), C) D) et E) du deuxième procédé sont identiques, respectivement aux étapes a), b), c) et d) du premier procédé.

Cependant, dans ce deuxième procédé, il est nécessaire de connaître la pression P₀ et la température T₀. C'est l'objet de l'étape A) qui n'est pas nécessaire dans le premier procédé selon l'invention.

En conséquence, l'étape D) du deuxième procédé diffère de l'étape c) du premier procédé, dans la mesure où la valeur de la pression, ou de la température ou à la fois de la pression et de la température doi(ven)t être(s) connue(s).

Par exemple, si l'on décide de ne faire varier que la pression, comme cela est envisageable avec le dispositif expérimental 100 décrit précédemment, la température peut être maintenue constante (T₀ = température de la chambre). En effet, il est important, pour ce deuxième procédé, de déterminer cette valeur de la variation de la pression (dans cet exemple) pour pouvoir mettre en œuvre l'étape F).

Si l'on revient à l'exemple concret décrit précédemment, on obtient, après avoir répété sept fois les étapes C) et D), la courbe de la figure 3 comportant huit paliers de pression.

L'étape F) peut être réalisée de la façon suivante.

On sait que l'indice optique *n_{G}* d'un milieu gazeux MG dépend de la température T (en °C), de la pression P (en Torr) et de la longueur d'onde λ (en µm) selon une relation du type : ${\left({n}_{G}-1\right)}_{TP} = {\left({n}_{G}-1\right)}_{λ} ∗ \frac{P}{720 , 775} ∗ \left[\frac{1 + P ∗ \left(0,817-0,0133∗T\right) ∗ {10}^{- 6}}{1 + 0 , 003661 ∗ T}\right]$ (R2) où :
*(n_{G} -* 1)*_{λ}* est une quantité représentative de l'indice optique *n_{G}* du milieu gazeux MG, à une température de 15°C et une pression de 1,013 bar (conditions standards), s'exprimant sous la forme : ${\left({n}_{G}-1\right)}_{λ} ∗ {10}^{- 8} = 8342 , 54 + \frac{2 406 147}{130 - {λ}^{2}} + \frac{15 998}{38 , 9 - {λ}^{2}}$ (R3)

Pour des variations de pression faibles, à savoir comprises entre 1 bar et quelques dizaines de bars, par exemple 50 bars, le terme quadratique en pression de la relation R2 ne contribue que très faiblement dans l'évolution de la quantité ${\left(\frac{\partial {\left({n}_{G}-1\right)}_{TP}}{\partial P}\right)}_{{C}_{i}}$*.* Par exemple, pour de l'air sec à une température d'environ 25°C et pour une variation de pression de 2 bars, la contribution de ce terme quadratique n'excède pas 0,1%. Toujours pour de l'air sec à une température d'environ 25°C et pour une variation de pression de 50 bars, la variation de ce terme quadratique n'excède pas quelques pourcents.

C'est pourquoi, on peut considérer, avec les données de la figure 3, correspondant à une variation totale de pression n'excédant pas 1 bar, que la quantité ${\left(\frac{\partial {\left({n}_{G}-1\right)}_{TP}}{\partial P}\right)}_{Ci}$ ne dépend pas de la pression. Autrement dit, on peut considérer que la quantité (*n_{G}* - 1)*_{TP}* évolue linéairement avec la pression.

Par ailleurs, à partir de la figure 3, on peut aussi noter que la variation de réflectivité R qui est mesurée évolue linéairement avec la pression puisque pour chaque capteur et chaque palier de pression, on obtient une valeur constante de cette variation de réflectivité mesurée.

De ce fait, pour chaque capteur, la quantité (*n_{G} -* 1)*_{TP}* évolue linéairement en fonction de la variation de réflectivité. Autrement dit, pour chaque capteur *Cᵢ* où i désigne l'indice du capteur avec 1 ≤ i ≤ N (N un entier naturel), on peut construire une relation du type : ${\left({n}_{G}-1\right)}_{{T}_{0} P , Ci} = {\left(\frac{\partial {\left({n}_{G}-1\right)}_{{T}_{0} P}}{\partial P}\right)}_{Ci} ∗ {\left(\frac{\partial R}{\partial P}\right)}_{Ci} ∗ {R}_{Ci} + {\left({n}_{G}-1\right)}_{{T}_{0} {P}_{0} , Ci}$ (R4) où :
la quantité ${\left(\frac{\partial R}{\partial P}\right)}_{Ci}$ provient de la régression linéaire effectuée, pour le capteur *Cᵢ*, à partir de la mesure en réflectivité R, en fonction de la pression P (dans l'exemple fourni, issu des données de la figure 3),
la quantité ${\left(\frac{\partial {\left({n}_{G}-1\right)}_{{T}_{0} P}}{\partial P}\right)}_{Ci}$ intervient, pour le capteur *Cᵢ*, pour normaliser la variation de réflectivité mesurée,
*R_{Ci}* est, pour le capteur *Cᵢ*, la variation de réflectivité mesurée (issu de la figure 3).

Dans l'exemple fourni, le milieu gazeux est de l'air sec à T = T₀ = 25°C et la longueur d'onde λ du faisceau lumineux FL est telle que λ = 632nm.

On en déduit, grâce aux relations R2 et R3 et compte tenu de l'approximation linéaire dans la gamme de pression considérée, que la quantité ${\left(\frac{\partial {\left({n}_{G}-1\right)}_{{T}_{0} P}}{\partial P}\right)}_{Ci} = {2,64 . 10}^{- 4}$ (avec les unités considérées).

De manière similaire, on en déduit également, par la relation que la quantité (*n_{G}* - 1)_{*T*0*P*0*,Ci*} = 0,000275545 (T₀ = 25°C et P₀ = 1,063 bar).

On obtient ainsi une relation R4 donnant, pour chaque capteur Cᵢ, l'évolution de l'indice optique local en fonction des données issues de la figure 3, à savoir ${\left(\frac{\partial R}{\partial P}\right)}_{Ci}$ et *R_{Ci}.*

S'agissant d'une calibration, chaque capteur fournit donc, selon la relation R4, des évolutions identiques de cet indice optique local en fonction de la pression du milieu gazeux.

On comprend que plus qu'une calibration relative, ce deuxième procédé permet d'obtenir également une calibration absolue des différents capteurs, dans la mesure où il permet d'obtenir l'évolution de l'indice optique conformément à la relation R4. En effet, une fois que les différentes évolutions d'indice optique pour les différents capteurs sont déterminées, la calibration du nez électronique est faite. Il ne reste alors qu'à la prendre en compte avant de débuter une mesure effective avec ce nez électronique.

Autrement dit, en mettant en œuvre ce deuxième procédé de calibration pour réaliser une calibration relative, on obtient également une calibration absolue.

S'il existe de légères différences, liées aux incertitudes de mesure, on peut ensuite choisir l'un de ces capteurs comme référence et appliquer la relation R4 obtenue pour ce capteur à l'ensemble des autres capteurs.

Pour cette deuxième méthode, et contrairement à la première méthode, il faut connaître le saut de pression avec précision (étape D)) pour pouvoir déterminer correctement, pour chaque capteur, l'évolution de l'indice optique local (étape F)).

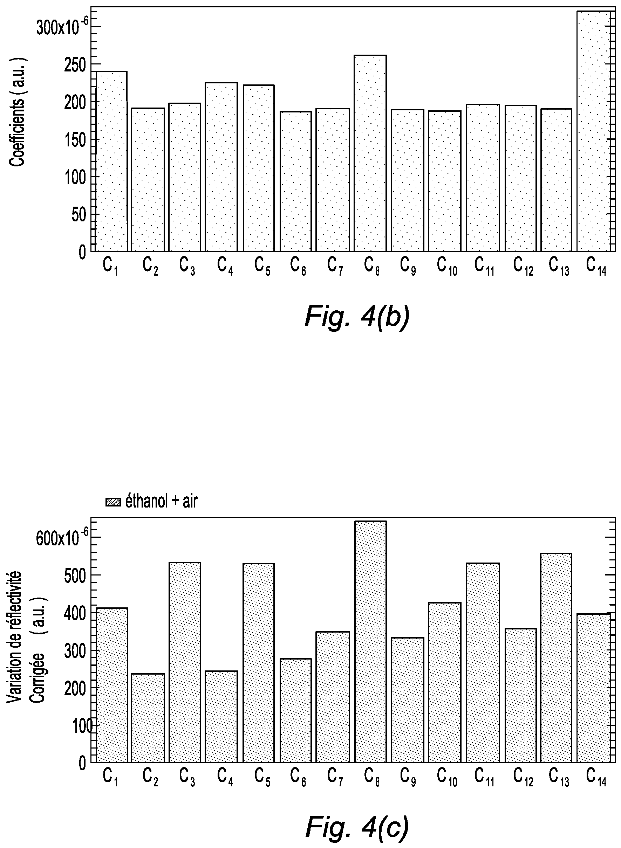

La figure 4 représente un test réalisé avec l'installation expérimentale 100 de la figure 1, dans les mêmes conditions que précédemment, à l'exception du nombre de capteurs retenus pour l'analyse (N = 14 capteurs) et de la nature du milieu gazeux MG. En effet, ici le milieu gazeux est de l'air sec chargé d'éthanol, à 200ppm. L'éthanol joue le rôle d'un analyte.

L'objectif de ce test est de montrer un cas d'application particulier, avec l'éthanol en tant qu'analyte.

La figure 4 comprend les figures 4(a) à 4(c).

La figure 4(a) représente, sous la forme d'un histogramme, la variation de réflectivité mesurée (données brutes - à comparer aux données de la figure 3).

On peut ensuite en déduire les facteurs correctifs, en se basant par exemple sur le capteur C₁ comme référence.

La figure 4(b) représente les facteurs correctifs pour chaque capteur. A ce stade, on connaît donc les facteurs correctifs conduisant à la calibration.

Enfin, la figure 4(c), représente la variation de réflectivité corrigée pour chaque capteur. Cette figure 4(c) correspond donc à la figure 4(a) corrigée par la figure 4(b). A ce stade, on est donc prêt pour débuter une mesure effective.

Avantageusement, et compte tenu de la sensibilité de l'appareil expérimental 100, on pourra avantageusement modifier la pression du milieu gazeux, à chaque saut de pression, d'une valeur comprise entre +10mbar et +2 bar, de préférence comprise entre +50mbar et +150mbar ou entre -10mbar et - 900mbar, de préférence entre -50mbar et -150mbar.

Par ailleurs, pour effectuer des mesures précises, il est avantageux d'effectuer une mesure en réflectivité (étape b) ou d) pour le premier procédé ou étape C) ou E) pour le deuxième procédé) réalisée sur une durée comprise entre 0,1s et 60mn, de préférence entre 1s et 10mn, puis moyennée. La durée de la mesure dépend de la précision souhaitée, mais aussi des caractéristiques du dispositif permettant de faire l'échantillonnage.

Comme cela a été réalisé dans l'exemple fourni, on répète avantageusement N fois les étapes c) et d) avant la mise en œuvre de l'étape e) ou selon le cas, on répète N fois les étapes D) et E) avant la mise en œuvre de l'étape F), avec N un entier naturel supérieur ou égal à 1.

Ainsi, à chaque répétition, la pression du milieu gazeux est différente d'une pression (ou, selon le cas, la température ou à la fois la pression et la température) pour laquelle une mesure a déjà été effectuée. Cela permet d'avoir plus de deux mesures et d'augmenter ainsi la qualité des mesures.

En tout état de cause, il est avantageux, à l'issue de la mesure en réflectivité et avant d'implémenter l'étape e) ou selon le cas l'étape F) de faire repasser la pression à la valeur initiale P₀ (ou selon le cas la température T à la valeur T0 ou à la fois la température et la pression) et ce, afin d'éliminer d'éventuelles dérives des signaux de mesure au cours de la prise de mesure. C'est d'ailleurs ce qui a été fait dans l'exemple fourni ici, où la dernière mesure est bien réalisée à la pression P₀ = 1,063bar (cf. figure 3).

Il convient de noter que les deux procédés décrits précédemment peuvent faire l'objet de variantes de réalisation.

En particulier, et comme on l'aura compris, on peut tout à fait réaliser des mesures en réflectivité (variation de réflectivité) en se basant sur une évolution de la température T du milieu gazeux MG, soit en maintenant la pression P₀ à une valeur constante la pression de ce milieu gazeux soit en faisant également varier la pression du milieu gazeux.

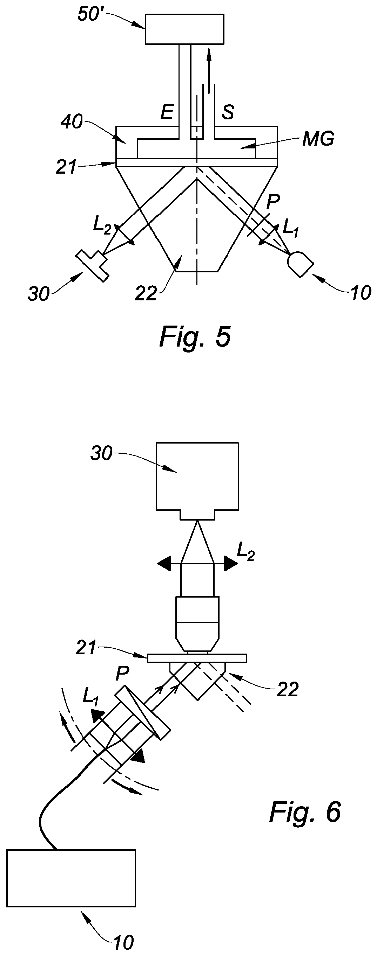

C'est ce qui est représenté sur la figure 5.

En comparaison à la figure 1, on note que l'installation expérimentale 100' inclut un dispositif 50' de régulation de la température, pour pouvoir faire évoluer la température. En pratique, ce dispositif peut se présenter sous la forme d'un fil électrique alimenté par le secteur pour réaliser un chauffage par effet Joule auquel est associé une boucle de régulation de la température.

Pour se fixer les idées, il convient de noter qu'une évolution de 10°C de la température du milieu gazeux MG correspond sensiblement à l'effet procuré par une évolution de pression de 100mbar. On peut s'appuyer sur la relation R1 à cet effet. Typiquement, on pourra donc prévoir, à chaque mesure, une évolution de température comprise entre +1°C et +100°C, de préférence entre +5°C et + 15°C ou entre -1°C et -50°C, de préférence entre -5°C et -15°C.

Bien entendu, le dispositif de régulation précité pourra être remplacé par un dispositif de régulation de la température et de la pression, lorsque l'on souhaite faire varier à la fois la température et la pression, ce dispositif étant par exemple une association des moyens décrits précédemment pour faire varier la température d'une part et la pression d'autre part.

En outre, qu'on se base (figure 1) sur une évolution de la pression du milieu gazeux (à température constante) ou (figure 5) sur une évolution de la température du milieu gazeux (à pression constante ou non), les procédés selon l'invention peuvent être mis en œuvre avec une mesure en transmittivité, à la place d'une mesure en réflectivité.

Sur la figure 6, on a ainsi représenté une installation expérimentale 100" permettant de mettre en œuvre une évolution de pression et/ou de température du milieu gazeux, avec une mesure en transmittivité. Pour des raisons de commodité, la pompe 50 et/ou, selon le cas, le dispositif 50' de régulation de température n'ont cependant pas été représentés sur cette figure 6, l'objectif étant simplement de représenter comment la mesure peut s'effectuer.

Plus généralement, un procédé conforme à l'invention peut être mis en œuvre avec une installation différente des installations 100, 100', 100", représentées respectivement sur les figures 1, 5 et 6.

En effet, pour toutes les installations décrites précédemment, on se base sur la résonance plasmon de surface (SPR) dans le cas d'une surface plane (couche métallique 21 posée sur un support 22 plan, en l'occurrence un prisme).

Or, l'homme du métier connaît bien d'autres installations qui permettent d'effectuer des mesures basées sur la résonance plasmon.

Nous citons ci-dessous, à titre non limitatif, quelques techniques envisageables.

Un procédé conforme à l'invention peut être mis en œuvre en utilisant la résonance plasmon de surface sur fibre optique, que ce soit en réflexion ou en transmission. Cette technique est par exemple présentée par Burgmeier & al., « Plasmonic nanoshelled functionalized etch fiber Bragg gratings for highly sensitive refractive index measurements », Optics Letters, vol. 40(4), pp. 546-549 (2015). Le dispositif proposé dans ce document est employé avec un milieu liquide, mais pourrait tout aussi bien être employé pour un milieu gazeux, donc pour un nez électronique.

Un procédé conforme à l'invention peut être mis en œuvre en utilisant la résonance plasmon de surface sur billes, que ce soit en réflexion ou en transmission. Cette technique est par exemple présentée, dans le cas d'une utilisation en réflexion par Frederix & al., « Biosensing based on light absorption on nanoscaled gold and silver nanoparticles », Anal. Chem. 2003, vol. 75, pp. 6894-6900. Le milieu diélectrique considéré est plutôt un liquide, mais peut être employé pour un milieu gazeux et donc un nez électronique.

Un procédé conforme à l'invention peut aussi être mis en œuvre en utilisant la résonance plasmon basée sur des nanocavités. Par exemple, l'article de Zhao Hua-Jin, "High sensitivity refractive index gas sensing enhanced by surface plasmon resonance with nano-cavity anteanna array ", 2012, Chinese Physical Society and IOP Publishing Ltd, Chinese Physics B, vol. 21 (8), pp. indique clairement que de tells nanocavités sont sensibles à un changement d'indice optique local. Cela peut donc être employé pour réaliser une calibration, par exemple pour un nez électronique.

En outre, l'utilisation de la résonance plasmon n'est pas la seule technique envisageable pour mettre en œuvre l'invention.

Ainsi, on peut envisager une technique de mesure basée sur la réfractométrie, pour mesurer une variation de l'indice optique. A cet effet, on peut utiliser un capteur optique de type « résonateur optique ». Dans ce cas, le résonateur remplit la fonction de la couche métallique d'un capteur à effet plasmon.

On pourra se référer à l'article de Luchansky & al. « High-Q optical sensors for chemical and biological analysis », Analytical chemistry, 2011, vo. 84 (2), pp. 793-821.

On pourra également se référer à l'article de Kim & al., « Integrated photonic glucose biosensor using a vertically coupled microring resonator in polymers », Optics Communications, 2008, vo. 281 (18), pp. 4644-4647 qui utilise cette propriété pour mesurer des indices optiques en milieux liquides.

On pourra encore se référer à Passaro et al., « Ammonia Optical Sensing by Microring Resonators », Sensors 2007, vol. 7, pp. 2741-2749 qui mesure des variations locales d'indice optique générées par l'ammoniac gazeux. En particulier, on note que le schéma de la page 7744 montre bien que la mesure se réalise par le changement de l'indice optique. En cela, elle peut donc être sensible a une variation de pression ou de température.

Dans les exemples fournis précédemment, le milieu gazeux d'intérêt utilisé pour faire de la calibration est de l'air sec. L'invention n'est pas limité à de l'air sec. En particulier, le milieu gazeux d'intérêt pour faire la calibration peut être, à titre non limitatif, l'air ambiant, un gaz porteur c'est-à-dire susceptible de porter un gaz à mesurer, tel que des composés organiques volatils (COV).

Par ailleurs, en pratique, l'obtention des facteurs correctifs peut se faire à la fabrication du nez électronique mais également par l'utilisateur dans le même environnement que ses mesures d'intérêt, ou lors d'une maintenance par exemple.

## Revendications

1. Procédé de calibration d'un nez électronique (20), ledit nez électronique comportant une pluralité de capteurs optiques (C₁ à C_{N}) agencés sur une surface (F1) et aptes à être en contact avec un milieu gazeux (MG) d'intérêt, lesdits capteurs optiques (C₁ à C_{N}) étant aptes à délivrer un signal représentatif de l'indice optique local du milieu gazeux (MG) d'intérêt lorsqu'ils sont excités par des photons, le procédé comprenant les étapes suivantes, après avoir placé le nez électronique (20) dans un milieu gazeux (MG) d'intérêt à la pression initiale P₀ et la température initiale T₀:
a) émettre des photons en direction des capteurs (C₁ à C_{N}) de façon à exciter lesdits capteurs;
b) mesurer le signal délivré par chacun des capteurs (C₁ à C_{N}), cette mesure fournissant autant de réponses qu'il y a de capteurs (C₁ à C_{N}) ;
c) modifier la pression et/ou la température du milieu gazeux d'intérêt ;
d) répéter l'étape b); et
le procédé étant **caractérisé en ce qu'**il comprend une étape :
e) calibrer le nez électronique en déterminant, pour chaque capteur (C₁ à C_{N}), un facteur correctif de telle sorte qu'une variation du signal entre les étapes d) et b) corrigée par ledit facteur correctif soit égale ou sensiblement égale à une variation du signal entre ces mêmes étapes pour une référence, cette référence étant fournie par un capteur de référence ou une combinaison de capteurs de référence.

2. Procédé de calibration d'un nez électronique (20) selon la revendication 1, dans lequel, préalablement à l'étape a), on détermine la pression initiale P₀ et/ou la température initiale T₀ du milieu gazeux (MG) d'intérêt.

3. Procédé de calibration d'un nez électronique (20) selon l'une des revendications précédentes, dans lequel la mesure réalisée à l'étape b) ou d), par exemple une mesure de réflectivité ou de transmittivité, s'effectue sur une durée comprise entre 0,1s et 60mn, de préférence entre 1s et 10mn, puis moyennée.

4. Procédé de calibration d'un nez électronique (20) selon l'une des revendications précédentes, dans lequel, avant de mettre en œuvre l'étape e), on répète N fois les étapes c) et d), avec N un entier naturel supérieur ou égal à 1, de sorte que la pression et/ou la température du milieu gazeux (MG) d'intérêt soit différente d'une pression et/ou d'une température du milieu gazeux (MG) d'intérêt pour lequel une mesure a déjà été effectuée.

5. Procédé de calibration d'un nez électronique (20) selon l'une des revendications précédentes, dans lequel, au cours de l'étape c), on modifie la pression et/ou la température du milieu gazeux (MG) d'intérêt vers une autre valeur connue.

6. Procédé de calibration d'un nez électronique (20) selon la revendication précédente, dans lequel à l'étape c) :
- on modifie la pression du milieu gazeux (MG) d'intérêt d'une valeur comprise entre +10mbar et + 2bar, de préférence entre +50mbar et +150mbar ou d'une valeur comprise entre -10mbar et -900mbar, de préférence entre -50mbar et - 150mbar ; et/ou
- on modifie la température du milieu gazeux (MG) d'intérêt d'une valeur comprise entre +1°C et +100°C, de préférence entre +5°C et +15°C ou entre -1°C et -50°C, de préférence entre -5°C et -15°C.

7. Procédé de calibration d'un nez électronique (20) selon la revendication 2 prise en combinaison avec l'une des revendications 5 ou 6, dans lequel, juste avant l'étape e), on met en œuvre une étape additionnelle consistant à modifier la pression et/ou la température du milieu gazeux (MG) d'intérêt à la pression initiale P₀ et/ou à la température initiale T₀.

8. Procédé de calibration d'un nez électronique (20) selon l'une des revendications précédentes, dans lequel le capteur optique (C₁ à C_{N}) est choisi parmi un capteur à effet plasmon, par exemple sur surface plane, fibre optique ou nanocavités ou un capteur susceptible de fonctionner par réfractométrie, par exemple un capteur à résonateur.

9. Procédé de calibration d'un nez électronique (20), ledit nez électronique comportant une pluralité de capteurs optiques (C₁ à C_{N}) agencés sur une surface (F1) et aptes à être en contact avec un milieu gazeux (MG) d'intérêt, lesdits capteurs optiques (C₁ à C_{N}) étant aptes à délivrer un signal représentatif de l'indice optique local du milieu gazeux (MG) d'intérêt lorsqu'ils sont excités par des photons, le procédé étant **caractérisé en ce qu'**il comprend les étapes suivantes, après avoir placé le nez électronique (20) dans un milieu gazeux (MG) d'intérêt à la pression initiale P₀ et la température initiale T₀ :
A) déterminer la pression initiale P₀ et la température initiale T₀ du milieu gazeux (MG) d'intérêt ;
B) émettre des photons en direction des capteurs optiques (C₁ à C_{N}) de façon à exciter lesdits capteurs ;
C) mesurer le signal délivré par chacun des capteurs optiques (C₁ à C_{N}), cette mesure fournissant autant de réponses qu'il y a de capteurs optiques (C₁ à C_{N}) ;
D) modifier la pression et/ou la température du milieu gazeux (MG) d'intérêt vers une autre ou des autres valeurs connues ;
E) répéter l'étape C); et
F) calibrer le nez électronique en calculant, pour chaque capteur (C₁ à C_{N}), l'évolution de l'indice optique du milieu gazeux (MG) d'intérêt à l'aide des mesures réalisées aux étapes A), C) et E).

10. Procédé de calibration d'un nez électronique (20) selon la revendication précédente, dans lequel, préalablement à l'étape A), on règle la pression et/ou la température du milieu gazeux (MG) à une valeur prédéterminée.

11. Procédé de calibration d'un nez électronique (20) selon l'une des revendications 9 ou 10, dans lequel la mesure réalisée à l'étape C) ou E), par exemple une mesure de réflectivité ou de transmittivité, s'effectue sur une durée comprise entre 0,1s et 60mn, de préférence entre 1s et 10mn, puis moyennée.

12. Procédé de calibration d'un nez électronique (20) selon l'une des revendications 9 à 11, dans lequel, avant de mettre en œuvre l'étape F), on répète N fois les étapes D) et E), avec N un entier naturel supérieur ou égal à 1, de sorte que la pression ou selon le cas, la température du milieu gazeux (MG) d'intérêt soit différente d'une pression ou selon le cas d'une température du milieu gazeux (MG) d'intérêt pour lequel une mesure a déjà été effectuée.

13. Procédé de calibration d'un nez électronique (20) selon l'une des revendications 9 à 12, dans lequel à l'étape D) :
- on modifie la pression du milieu gazeux (MG) d'intérêt d'une valeur comprise entre +10mbar et + 2bar, de préférence entre +50mbar et +150mbar ou d'une valeur comprise entre -10mbar et -900mbar, de préférence entre -50mbar et - 150mbar ; et/ou
- on modifie la température du milieu gazeux (MG) d'intérêt d'une valeur comprise entre +1°C et +100°C, de préférence entre +5°C et +15°C, ou entre -1°C et - 50°C, de préférence entre -5°C et -15°C.

14. Procédé de calibration d'un nez électronique selon l'une des revendications 9 à 13, dans lequel, juste avant l'étape F), on met en œuvre une étape additionnelle consistant à modifier la pression et/ou la température du milieu gazeux d'intérêt à la pression initiale P₀ et/ou la température initiale T₀.

15. Procédé de calibration d'un nez électronique (20) selon l'une des revendications 9 à 14, dans lequel le capteur optique (C₁ à C_{N}) est choisi parmi un capteur à effet plasmon, par exemple sur surface plane, fibre optique ou nanocavités, ou un capteur susceptible de fonctionner par réfractométrie, par exemple un capteur à résonateur.

## Patentansprüche

1. Verfahren zum Kalibrieren einer elektronischen Nase (20), wobei die besagte elektronische Nase eine Vielzahl von optischen Sensoren (C₁ bis C_{N}) umfasst, die auf einer Oberfläche (F1) angeordnet und geeignet sind, mit einem relevanten Gasmedium (MG) in Kontakt zu sein, wobei die besagten optischen Sensoren (C₁ bis C_{N}) geeignet sind, ein Signal herauszugeben, das den lokalen optischen Index des besagten relevanten Gasmediums (MG) repräsentiert, wenn sie durch Photonen angeregt werden, wobei das Verfahren die folgenden Schritte umfasst, nachdem die besagte elektronische Nase (20) in einem besagten relevanten Gasmedium (MG) bei dem Anfangsdruck P₀ und der Anfangstemperatur T₀ platziert wurde:
a) Emittieren von Photonen in Richtung der Sensoren (C₁ bis C_{N}) um die besagten Sensoren anzuregen;
b) Messen des von jedem der besagten Sensoren (C₁ bis C_{N}) herausgegebenen Signals, wobei diese Messung so viele Antworten liefert, wie es besagte Sensoren (C₁ bis C_{N}) gibt;
c) Ändern des Drucks und/oder der Temperatur des relevanten Gasmediums;
d) Wiederholen des Schritts b); und
wobei das Verfahren **dadurch gekennzeichnet ist, dass** es einen folgenden Schritt umfasst:
e) Kalibrieren der besagten elektronischen Nase durch Bestimmen eines Korrekturfaktors für jeden besagten Sensor (C₁ bis C_{N}), so dass eine Variation des Signals zwischen den Schritten d) und b), die durch den besagten Korrekturfaktor korrigiert wird, gleich oder im Wesentlichen gleich einer Variation des Signals zwischen denselben Schritten für eine Referenz ist, wobei die besagte Referenz durch einen besagten Referenzsensor oder eine besagte Kombination von Referenzsensoren bereitgestellt wird.

2. Verfahren zum Kalibrieren einer elektronischen Nase (20) nach Anspruch 1, wobei vor Schritt a) der Anfangsdruck P₀ und/oder die Anfangstemperatur T₀ des relevanten Gasmediums (MG) bestimmt wird.

3. Verfahren zum Kalibrieren einer elektronischen Nase (20) nach einem der vorstehenden Ansprüche, wobei die bei Schritt b) oder d) durchgeführte Messung, beispielsweise eine Messung des Reflexionsvermögens oder des Transmissionsvermögens, über einen Zeitraum zwischen 0,1 s und 60 min, bevorzugt zwischen 1 s und 10 min, durchgeführt und dann gemittelt wird.

4. Verfahren zum Kalibrieren einer elektronischen Nase (20) nach einem der vorstehenden Ansprüche, wobei vor dem Durchführen von Schritt e) die Schritte c) und d) N-mal wiederholt werden, wobei N eine natürliche Zahl größer oder gleich 1 ist, so dass der Druck und/oder die Temperatur des relevanten Gasmediums (MG) sich von einem Druck und/oder einer Temperatur des relevanten Gasmediums (MG) unterscheidet, für die bereits eine Messung durchgeführt wurde.

5. Verfahren zum Kalibrieren einer elektronischen Nase (20) nach einem der vorstehenden Ansprüche, wobei während Schritt c) der Druck und/oder die Temperatur des relevanten Gasmediums (MG) auf einen anderen bekannten Wert geändert wird.

6. Verfahren zum Kalibrieren einer elektronischen Nase (20) nach dem vorstehenden Anspruch, wobei bei Schritt c):
- der Druck des relevanten Gasmediums (MG) um einen Wert zwischen +10 mbar und +2 bar, bevorzugt zwischen +50 mbar und +150 mbar, oder um einen Wert zwischen -10 mbar und -900 mbar, bevorzugt zwischen -50 mbar und -150 mbar, geändert wird; und/oder
- die Temperatur des relevanten Gasmediums (MG) um einen Wert zwischen +1 °C und +100 °C, bevorzugt zwischen +5 °C und +15 °C, oder zwischen -1 °C und -50 °C, bevorzugt zwischen -5 °C und -15 °C, geändert wird.

7. Verfahren zum Kalibrieren einer elektronischen Nase (20) nach Anspruch 2 in Kombination mit einem der Ansprüche 5 oder 6, wobei unmittelbar vor Schritt e) ein zusätzlicher Schritt durchgeführt wird, der darin besteht, den Druck und/oder die Temperatur des relevanten Gasmediums (MG) auf den Anfangsdruck P₀ und/oder die Anfangstemperatur T₀ zu ändern.

8. Verfahren zum Kalibrieren einer elektronischen Nase (20) nach einem der vorstehenden Ansprüche, wobei der optische Sensor (C₁ bis C_{N}) ausgewählt ist aus einem Sensor mit Plasmoneneffekt, beispielsweise auf ebener Oberfläche, Lichtleiter oder Nanokavitäten, oder einem Sensor, der durch Refraktometrie betrieben werden kann, beispielsweise einem Resonatorsensor.

9. Verfahren zum Kalibrieren einer elektronischen Nase (20), wobei die besagte elektronische Nase eine Vielzahl von optischen Sensoren (C₁ bis C_{N}) umfasst, die auf einer Oberfläche (F1) angeordnet und geeignet sind, mit einem relevanten Gasmedium (MG) in Kontakt zu sein, wobei die besagten optischen Sensoren (C₁ bis C_{N}) geeignet sind, ein Signal herauszugeben, das den lokalen optischen Index des besagten relevanten Gasmediums (MG) repräsentiert, wenn sie durch Photonen angeregt werden, wobei das Verfahren **dadurch gekennzeichnet ist, dass** es die folgenden Schritte umfasst, nachdem die besagte elektronische Nase (20) in einem besagten relevanten Gasmedium (MG) bei dem Anfangsdruck P₀ und der Anfangstemperatur T₀ platziert wurde:
A) Bestimmen des Anfangsdrucks P₀ und der Anfangstemperatur T₀ des relevanten Gasmediums (MG);
B) Emittieren von Photonen in Richtung der optischen Sensoren (C₁ bis C_{N}) um die besagten Sensoren anzuregen;
C) Messen des von jedem der optischen Sensoren (C₁ bis C_{N}), herausgegebenen Signals, wobei diese Messung so viele Antworten liefert, wie es optische Sensoren (C₁ bis C_{N}) gibt;
D) Ändern des Drucks und/oder der Temperatur des relevanten Gasmediums (MG) auf einen oder mehrere andere bekannte Werte;
E) Wiederholen von Schritt C); und
F) Kalibrieren der elektronischen Nase durch Berechnen der Änderung des optischen Index des relevanten Gasmediums (MG) für jeden Sensor (C₁ bis C_{N}) anhand der in den Schritten A), C) und E) durchgeführten Messungen.

10. Verfahren zum Kalibrieren einer elektronischen Nase (20) nach dem vorstehenden Anspruch, wobei vor Schritt A) der Druck und/oder die Temperatur des Gasmediums (MG) auf einen vorbestimmten Wert eingestellt wird.

11. Verfahren zum Kalibrieren einer elektronischen Nase (20) nach einem der Ansprüche 9 oder 10, wobei die bei Schritt C) oder E) durchgeführte Messung, beispielsweise eine Messung des Reflexionsvermögens oder des Transmissionsvermögens, über einen Zeitraum zwischen 0,1 s und 60 min, bevorzugt zwischen 1 s und 10 min, durchgeführt und dann gemittelt wird.

12. Verfahren zum Kalibrieren einer elektronischen Nase (20) nach einem der Ansprüche 9 bis 11, wobei vor dem Durchführen von Schritt F) die Schritte D) und E) N-mal wiederholt werden, wobei N eine natürliche Zahl größer oder gleich 1 ist, so dass der Druck oder gegebenenfalls die Temperatur des relevanten Gasmediums (MG) sich von einem Druck oder gegebenenfalls einer Temperatur des relevanten Gasmediums (MG) unterscheidet, für die bereits eine Messung durchgeführt wurde.

13. Verfahren zum Kalibrieren einer elektronischen Nase (20) nach einem der Ansprüche 9 bis 12, wobei bei Schritt D):
- der Druck des relevanten Gasmediums (MG) um einen Wert zwischen +10 mbar und +2 bar, bevorzugt zwischen +50 mbar und +150 mbar, oder um einen Wert zwischen -10 mbar und -900 mbar, bevorzugt zwischen -50 mbar und -150 mbar, geändert wird; und/oder
- die Temperatur des relevanten Gasmediums (MG) um einen Wert zwischen +1 °C und +100 °C, bevorzugt zwischen +5 °C und +15 °C, oder zwischen -1 °C und -50 °C, bevorzugt zwischen -5 °C und -15 °C, geändert wird.

14. Verfahren zum Kalibrieren einer elektronischen Nase nach einem der Ansprüche 9 bis 13, wobei unmittelbar vor Schritt F) ein zusätzlicher Schritt durchgeführt wird, der darin besteht, den Druck und/oder die Temperatur des relevanten Gasmediums auf den Anfangsdruck P₀ und/oder die Anfangstemperatur T₀ zu ändern.

15. Verfahren zum Kalibrieren einer elektronischen Nase (20) nach einem der Ansprüche 9 bis 14, wobei der optische Sensor (C₁ bis C_{N}) ausgewählt ist aus einem Sensor mit Plasmoneneffekt, beispielsweise auf ebener Oberfläche, Lichtleiter oder Nanokavitäten, oder einem Sensor, der durch Refraktometrie betrieben werden kann, beispielsweise einem Resonatorsensor.

## Claims

1. A method for calibrating an electronic nose (20), said electronic nose comprising a plurality of optical sensors (C₁ à C_{N}) arranged on a surface (F1) and capable of being in contact with a gaseous medium (MG) of interest, said optical sensors (C₁ à C_{N}) being capable of delivering a signal representative of the local optical index of the gaseous medium (MG) of interest when they are excited by photons, the method comprising the following steps, after having placed the electronic nose (20) in a gaseous medium (MG) of interest at the initial pressure P₀ and the initial temperature T₀:
a) emitting photons in the direction of the sensors (C₁ à C_{N})so as to excite said sensors;
b) measuring the signal delivered by each of the sensors (C₁ à C_{N}), this measurement providing as many responses as there are sensors (C₁ à C_{N});
c) modifying the pressure and/or temperature of the gaseous medium of interest;
d) repeating step b); and
the method being **characterized in that** it comprises a step:
e) calibrating the electronic nose by determining, for each sensor (C₁ à C_{N}), a correction factor such that a variation in the signal between steps d) and b) corrected by the correction factor is equal or substantially equal to a variation in the signal between these same steps for a reference, said reference being provided by a reference sensor or a combination of reference sensors.

2. The method for calibrating an electronic nose (20) according to claim 1, wherein, prior to step a), the initial pressure P₀ and/or the initial temperature T₀ of the gaseous medium (MG) of interest is determined.

3. The method for calibrating an electronic nose (20) according to one of the preceding claims, wherein the measurement performed in step b) or d), for example a reflectivity or transmittivity measurement, is carried out over a period of between 0.1s and 60mn, preferably between 1s and 10mn, then averaged.

4. The method for calibrating an electronic nose (20) according to one of the preceding claims, wherein, before implementing step e), steps c) and d) are repeated N times, with N a natural integer greater than or equal to 1, so that the pressure and/or temperature of the gaseous medium (MG) of interest is different from a pressure and/or temperature of the gaseous medium (MG) of interest for which a measurement has already been made.

5. The method for calibrating an electronic nose (20) according to one of the preceding claims, wherein, in step c), the pressure and/or temperature of the gaseous medium (MG) of interest is modified to another known value.

6. The method for calibrating an electronic nose (20) according to the preceding claim, wherein in step c):
- the pressure of the gaseous medium (MG) of interest is modified by a value between +10mbar and +2bar, preferably between +50mbar and +150mbar or by a value between -10mbar and -900mbar, preferably between -50mbar and -150mbar ; and/or
- the temperature of the gaseous medium (MG) of interest is modified by a value between +1°C and +100°C, preferably between +5°C and +15°C or between -1°C and -50°C, preferably between -5°C and -15°C.

7. The method for calibrating an electronic nose (20) according to one of claims 5 or 6, wherein, just before step e), an additional step is implemented consisting in modifying the pressure and/or temperature of the gaseous medium (MG) of interest to the initial pressure P₀ and/or initial temperature T₀.

8. The method for calibrating an electronic nose (20) according to one of the preceding claims, wherein the optical sensor is selected from a plasmon effect sensor (C₁ à C_{N}), for example on a flat surface, optical fiber or nanocavities or a sensor capable of operating by refractometry, for example a resonator sensor.

9. A method for calibrating an electronic nose (20), said electronic nose comprising a plurality of optical sensors (C₁ à C_{N}) arranged on a surface (F1) and capable of being in contact with a gaseous medium (MG) of interest, said optical sensors (C₁ à C_{N}) being capable of delivering a signal representative of the local optical index of the gaseous medium (MG) of interest when excited by photons, the method being **characterized in that** it comprises the following steps, after having placed the electronic nose (20) in a gaseous medium (MG) of interest at the initial pressure P₀ and the initial temperature T₀:
A) determining the initial pressure P₀ and the initial temperature T₀ of the gaseous medium (MG) of interest;
B) emitting photons in the direction of the optical sensors (C₁ à C_{N}) so as to excite said sensors;
C) measuring the signal delivered by each of the optical sensors (C₁ à C_{N}), this measurement providing as many responses as there are optical sensors (C₁ à C_{N});
D) modifying the pressure and/or temperature of the gaseous medium (MG) of interest to another or other known values;
E) repeating step C); and
F) calibrating the electronic nose (20) by calculating, for each sensor (C₁ à C_{N}), the evolution of the optical index of the gaseous medium (MG) of interest using the measurements made in steps A), C) and E).

10. The method for calibrating an electronic nose (20) according to the preceding claim, wherein, prior to step A), the pressure and/or temperature of the gaseous medium (MG) is adjusted to a predetermined value.

11. The method for calibrating an electronic nose (20) according to one of claims 9 or 10, wherein the measurement performed in step C) or E), for example a reflectivity or transmittivity measurement, is carried out over a period of between 0,1s and 60mn, preferably between 1s and 10mn, and then averaged.

12. The method for calibrating an electronic nose (20) according to one of claims 9 to 11, wherein, before implementing step F), steps D) and E) are repeated N times, with N a natural integer greater than or equal to 1, so that the pressure or, as appropriate, the temperature of the gaseous medium (MG) of interest is different from a pressure or, as appropriate, a temperature of the gaseous medium (MG) of interest for which a measurement has already been made.

13. The method for calibrating an electronic nose (20) according to one of claims 9 to 12, wherein in step D):
- the pressure of the gaseous medium (MG) of interest is modified by a value between +10mbar and +2bar, preferably between +50mbar and +150mbar or by a value between - 10mbar and -900mbar, preferably between -50mbar and -150mbar; and/or
- the temperature of the gaseous medium (MG) of interest is modified by a value between +1°C and +100°C, preferably between +5°C and +15°C, or between -1°C and -50°C, preferably between -5°C and -15°C.

14. The method for calibrating an electronic nose according to one of claims 9 to 13, wherein, just before step F), an additional step is implemented consisting in modifying the pressure and/or temperature of the gaseous medium of interest at the initial pressure P₀ and/or initial temperature T₀.

15. The method for calibrating an electronic nose (20) according to one of claims 9 to 14, wherein the optical sensor (C₁ à C_{N}) is selected from a plasmon effect sensor, for example on a flat surface, optical fiber or nanocavities, or a sensor capable of operating by refractometry, for example a resonator sensor.
